(19) Europäisches Patentamt
European Patent Office
Office européen des brevets

(11) **EP 3 554 339 B1**

(12) **EUROPEAN PATENT SPECIFICATION**

(45) Date of publication and mention
of the grant of the patent:
**10.04.2024   Bulletin 2024/15**

(21) Application number: **17818612.8**

(22) Date of filing: **19.12.2017**

(51) International Patent Classification (IPC):
**A61B 5/00** *(2006.01)*      **A61B 5/05** *(2021.01)*

(52) Cooperative Patent Classification (CPC):
**A61B 5/0507; A61B 5/708; A61B 5/7264;**
A61B 2562/0228; A61B 2562/046; G16H 50/20

(86) International application number:
**PCT/GB2017/053825**

(87) International publication number:
**WO 2018/115858 (28.06.2018 Gazette 2018/26)**

(54) **A MEDICAL IMAGING SYSTEM**

SYSTEM ZUR MEDIZINISCHEN BILDGEBUNG

SYSTÈME D'IMAGERIE MÉDICALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.12.2016   GB 201621659**

(43) Date of publication of application:
**23.10.2019   Bulletin 2019/43**

(73) Proprietor: **Micrima Limited
Bristol BS2 0EL (GB)**

(72) Inventors:
• **BORE, Chris Frank
Bristol BS2 0EL (GB)**
• **BANNISTER, Peter Romilly
Bristol BS2 0EL (GB)**
• **IRIARTE, Ana
Bristol BS2 0EL (GB)**
• **GIBBINS, David Rhys
Bristol BS2 0EL (GB)**
• **TSUI, Chun Sing Louis
Bristol BS2 0EL (GB)**

(74) Representative: **Haseltine Lake Kempner LLP
One Portwall Square
Portwall Lane
Bristol BS1 6BH (GB)**

(56) References cited:
**US-A1- 2006 241 409     US-A1- 2012 190 977
US-A1- 2016 106 334**

## Description

The invention relates to a medical imaging system for tissue identification, *in vivo.*

Various medical imaging techniques are known for examining the human body. Such imaging techniques may be used to interrogate tissues and organs in order to identify tissue. For example, X-ray (mammography), microwave imaging, ultrasound, MRI are all common imaging modalities. Such techniques are commonly used to examine breast tissue, but may also be used in other areas of the body, such as the liver, pancreas, prostate, thyroid, lungs, ovaries and lymph nodes. Known examples of medical imaging techniques are described in US 2016/106334, US 2006/241409 and US 2012/190977.

With many existing techniques, once a region of interest has been identified in which unidentified tissue exists, it is necessary to take a biopsy from the site and perform further tests on the biopsy.

It is desirable to provide an enhanced imaging technique which is able to provide further information regarding a region of interest such that a biopsy is not required or is only required in some circumstances.

In accordance with an aspect of the invention, there is provided a microwave imaging system comprising:

a microwave antenna array comprising a transmitting antenna and a plurality of receiving antennas, wherein the transmitting antenna is configured to transmit microwave signals over a range of frequencies so as to illuminate a body part of a patient and the receiving antennas are configured to receive the microwave signals following scattering within the body part and acquire data representing the received microwave signals affected by scattering arising from objects within the body part; and
a processor configured to:

process the acquired data to determine a plurality of sets of scattering parameters for a plurality of points within the body part, wherein each set of scattering parameters represents the scattered field, at respective frequencies across the range of frequencies, from a corresponding point within the body part;

generate an output indicative of the internal structure of the body part, using the plurality of sets of scattering parameters, to identify a region of interest within the body part; and

classify the region of interest based on a type of tissue therein by analysing the change of the scattering parameters, in a set of scattering parameters corresponding to the region of interest, over the range of frequencies.

The scattering ptest2arameters may be frequency domain scattering parameters. For example, a scattering parameter for a point (or region of interest) is determined for each frequency and may be considered to form a spectrum across the range of frequencies.

The processor may classify the region of interest as malignant or benign.

The processor may classify the region of interest based on an average magnitude value of the scattering parameters in the set of scattering parameters corresponding to the region of interest over a first frequency range and on an average magnitude value of the scattering parameters in the set of scattering parameters corresponding to the region of interest over a second frequency range.

The processor may classify the region of interest based on a decision boundary dividing values for the first and second frequency ranges into at least two classes.

The first and second frequency ranges may be non-adjacent.

Analysing the change of the scattering parameters in the set of scattering parameters corresponding to the region of interest over the range of frequencies may comprise determining a change in permittivity with frequency based on the change of the scattering parameters in the set of scattering parameters over the range of frequencies; and the processor may be configured to classify the region of interest based on the determined change in permittivity with frequency.

Analysing the change of the scattering parameters in the set of scattering parameters corresponding to the region of interest over the range of frequencies may comprise determining the static conductivity and/or the polarisation intensity of the region of interest, based on the change in scattering parameters in the set of scattering parameters over the range of frequencies; and the processor may be configured to classify the region of interest based on a predetermined correlation between tissue type and the static conductivity and/or the polarisation intensity.

The processor may be configured to project the scattering parameters in the set of scattering parameters corresponding to the region of interest (e.g. the average scattering parameters for the first and second frequency ranges) to a predefined reference coordinate space; and the processor may be configured to classify the region of interest based on the position of the projected scattering parameters within the predefined reference coordinate space.

The predefined reference coordinate space may comprise a plurality of axes, wherein each axis corresponds to a principal component of a set of reference data.

The set of reference data may comprise a plurality of sets of scattering parameters obtained for respective sets of sample tissues of known tissue types. For example, a plurality of sets of scattering parameters may have been obtained using the method of the invention for sample tissues of known tissue types.

The microwave antenna array may be formed on a substrate, wherein the substrate is contoured or contourable to conform to the body part.

The substrate may be hemispherical.

The processor may be further configured to generate an image of the internal structure of the breast based on the plurality of sets of scattering parameters.

In accordance with an aspect not falling within the scope of the invention as claimed, there is provided a medical imaging method comprising:

illuminating a body part of a patient with microwave signals emitted by a transmitting antenna of a microwave antenna array over a range of frequencies;

receiving the microwave signals following scattering within the body part at a plurality of receiving antennas of the microwave antenna array;

processing the scattered microwave signals to obtain a set of scattering parameters for the microwave signals over the range of frequencies and generating an output indicative of the internal structure of the body part;

identifying a region of interest within the body part from the output; and

classifying the region of interest based on a type of tissue therein by analysing the change of the scattering parameters for the region of interest over the range of frequencies.

For a better understanding of the invention, and to show more clearly how it may be carried into effect, reference will now be made, by way of example, to the accompanying drawings, in which:-

Figure 1 is a system diagram of a medical imaging system according to an embodiment of the invention;

Figure 2 is a schematic view of the medical imaging system;

Figure 3 is a flowchart depicting a sampling method;

Figure 4 is a flowchart depicting a classification method according to an embodiment of the present invention;

Figure 5 is a flowchart of a model-based classifier method;

Figure 6 is a graph of high frequency magnitude against low frequency magnitude;

Figure 7 is a graph of medium frequency magnitude against low frequency magnitude; and

Figure 8 illustrates a method of determining a classifier based on a principal component analysis (PCA).

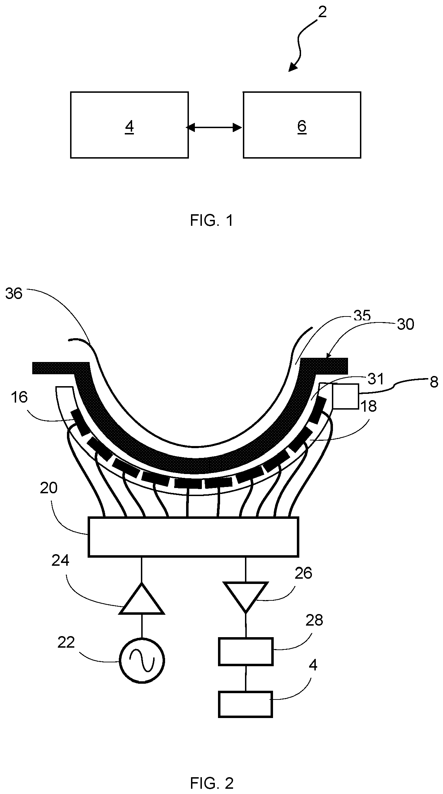

Figure 1 shows a medical imaging system 2 according to an embodiment of the invention. The medical imaging system generally comprises a processor 4 and a microwave antenna array 6 in communication with the processor 4.

As shown in Figure 2, the antenna array 6 comprises a plurality N of antennas 16 which are arranged over the surface of, or within, a shell substrate 18. The shell 18 has a curved profile as shown. In particular, the shell 18 is part or hemi-spherical and is configured to approximate the shape of a breast. The antennas 16 are arranged over the shell 18 such that they all point to a common focal point.

The antennas 16 are each electrically connected to a switching matrix 20. The switching matrix 20 is in turn connected to both a transmit path and a receive path. The transmit path comprises a signal generator 22 coupled to an amplifier 24. The receive path comprises an amplifier 26 coupled to a detector 28 and the processor 4.

The switching matrix 20 selectively couples each of the antennas 16 to either the transmit path or the receive path.

The antenna array 6 is operated in a multi-static fashion. Specifically, the switching matrix 20 is controlled so as to connect one of the antennas 16 to the transmit path and the remaining antennas 16 to the receive path. The signal generator 22 generates a stepped frequency continuous wave (CW) signal which is amplified by the amplifier 24 and then transmitted by the antenna 16 connected to the transmit path. The stepped frequency continuous wave signal is a sequential series of pulses of continuous wave energy, where each pulse has its frequency stepped up across a range of frequencies, typically within the 3-8 GHz range. The other antennas 16 receive the transmitted signal and the received signal is detected and then recorded by the processor 4.

As shown in Figure 2, the shell 18 receives a cup 30. The cup 30 has a complementary shape to the shell 18 such that it fits within the shell 18. A layer of coupling fluid (dielectric constant controlled fluid) may be inserted in the gap 31 between the shell 18 and the cup 30 so as to improve the coupling between the antennas 16 and the cup 30 in order to minimise signal loss and thus improve transmission of the microwave signal.

An actuator (not shown), such as a motor, may be connected to the microwave antenna array 6. The actuator is configured to move the microwave antenna array 6 relative to the cup 30 which remains stationary against the breast 36. Specifically, the actuator causes the microwave antenna array 6 to rotate relative to the cup 30 about the breast 36. The microwave antenna array 6 rotates about the center of the shell 18 (i.e. its axis of symmetry). This may be enabled by a threaded engagement between the cup 30 and the shell 18. In particular, the outside of the cup 30 and the inside of the shell 18 may have threaded portions which engage to allow the shell 18 and the antenna array 6 to be rotated relative to the cup 30. This may also allow the cup 30 to be quickly and easily removed so as to enable the coupling fluid to be replaced.

The antennas 16 may be as described in WO 2009/060181. In particular, the antennas 16 may comprise a slot 16 formed in a conductive element, the slot having a rectangular external boundary defined by a substantially closed internal edge of the conductive element. A microstrip feed line may be spaced from the conductive element by a dielectric substrate with the distal end of the line positioned at the geometric centre of the slot.

In use, a patient lies in a prone position such that their breast 36 sits in the cup 30. A layer of coupling fluid may also be provided in the gap 35 between the cup 30 and the breast 36 in order to improve coupling between the antennas 16 and the breast 36.

Although not shown, one or more inserts may be placed inside the cup 30 so as to enable a better fit between the internal surface of the cup 30 and the breast 36. For example, a plurality of such inserts may be provided, each having different shapes and sizes, to enable the system to be better adapted to breasts of different shapes and sizes. The inserts may be made from the same material as the cup (e.g. ceramic).

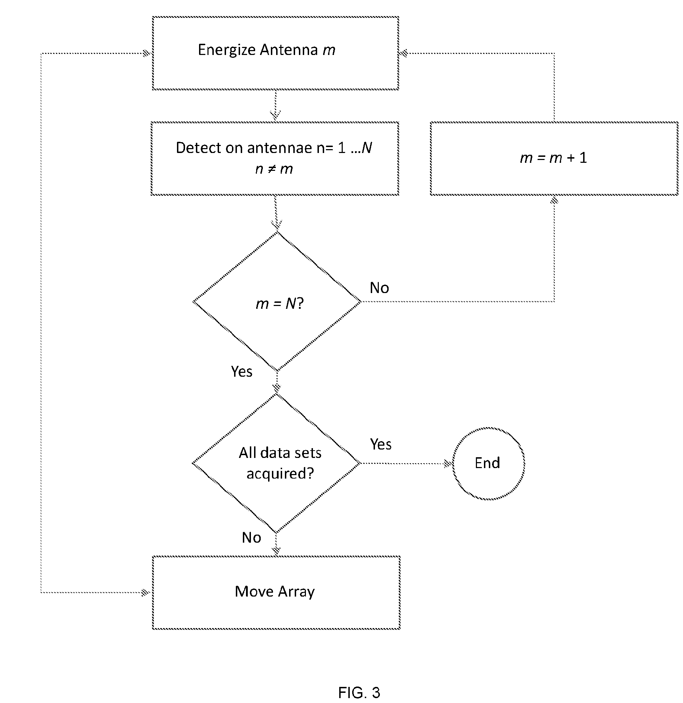

Figure 3 shows a flowchart of a data acquisition method. As shown, the switching matrix 20 connects an antenna m of the N antennas to the transmit path. All other antennas 16 (n = 1...N, n≠m) are connected to the receive path. The antenna 16 connected to the transmit path illuminates the breast 36 with the microwave signal. The signal is scattered by the breast tissue and the scattered signal is received at each of the non-transmitting antennas 16 where it is detected (possibly in a time-sharing arrangement) and recorded. If m≠N, the switching matrix 20 steps to the next antenna 16 (m = m + 1) to be connected to the transmit path. This is repeated until all antennas 16 have been connected to the transmit path.

If additional data sets are required, then the actuator is used to rotate the array 6 relative to the breast 36 while retaining the breast in position. The acquisition process is then repeated with the antenna array 6 in the new configuration.

The processor 4 may record the relative difference between the measured phase and amplitude of the transmitted signal as compared to the phase and amplitude of the scattered signal, recorded as a complex number (having real and imaginary parts).

The signal detected at each antenna 16 is affected by scattering arising from objects within the imaged volume (i.e. the breast 36). In particular, tumours can generate significant reflections as they exhibit higher dielectric properties than adipose and connective tissues due to their significant water content and so can be identified in the acquired data.

The acquired data may be used by the processor 4 to determine scattering parameters that represent the (e.g. magnitude of the) scattered field at respective frequencies across the range of frequencies, from a point within the body part.

The acquired data may be used by the processor 4 to construct an image of the internal structure of the breast 36. For example, scattering parameters (over the range of frequencies) may be determined for a plurality of (and in embodiments all of the resolvable) points within the body part, e.g. breast, and used to generate an image of the breast (or rather to generate data that could be used to represent the internal structure of the body part (breast) in an image). From this data, the processor 4 is able to identify (possibly, with additional user input or confirmation) a region of interest (if present), e.g. a region which is to be classified. This may be a region in which a possible tumour or other pathology may exist.

The above described processing of the acquired data (i.e. the Data reconstruction) may be performed using Phased Array (frequency domain), Delay and Sum (DAS - time domain) techniques or any other suitable technique, such as 3D Fourier Transformation, Back Projection, etc.

It has been found that the dielectric properties of the breast 36, and thus the scattering parameters measured by the processor 4 for a region or point of interest, are frequency-dependent. Further, it has been found that the frequency response of the dielectric properties varies for different types of tissue. This allows the tissue within a region of interest to be identified, and for the tissue to be further classified as being either malignant or benign, for example, based directly on the scattering parameters used to identify the region. That is, the tissue within a region of interest may be classified based on an analysis of the scattering parameters for the region of interest over the range of frequencies.

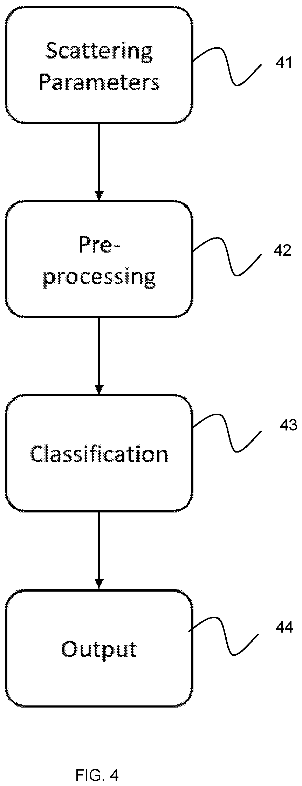

The overall classification procedure will now be described with respect to Figure 4, which is a flowchart illustrating the various processing steps according to an embodiment of the invention.

As can be seen in Figure 4, the method begins at step 41 where a set of scattering parameters is determined for the body part in a manner as described above with respect to Figure 3. This step will also include using the scattering parameters to generate an output indicative of the internal structure of the body part to identify a region of interest within the body part.

After a region of interest within the body part and its corresponding set of scattering parameters have been identified, the method of the invention will proceed to step 42, at which point the set of scattering parameters for the region of interest is processed and analysed pre-classification. In particular, the processor 4 will process the set of scattering parameters to determine information that is indicative of and/or derived from the change of the scattering parameters over the range of frequency.

This information is then used at step 43 in order to classify the tissue in the region of interest based on a comparison with a "classifier" that has been trained on data obtained for known tissue types. The classification may include identifying the type of tissue in the region of interest (as e.g. skin, muscle, fat, adipose etc.), and/or identifying the tissue as being either malignant or benign.

At step 44, the result of the classification may be output to aid diagnosis by a clinician. The output may take the form of a tissue identification (e.g. an identification that the tissue is skin, muscle or fat etc.), a binary decision (e.g. malignant/benign), or a likelihood indication (e.g. an indication that the tissue is 90% likely to be benign).

It will be appreciated that any suitable and desired pre-classification processing method may be used at step 42 in order to process the set of scattering parameters, and that the specific processing that is performed on the set of scattering parameters will depend on the type of classifier that has been trained and that is to be used at step 43.

For ease of explanation, the various pre-classification processing steps that may be used in accordance with the present invention will now be described separately in the following sections. However, it will be appreciated that any one or more of the methods described herein can be used in isolation or in combination, as appropriate.

## Model-based classifier

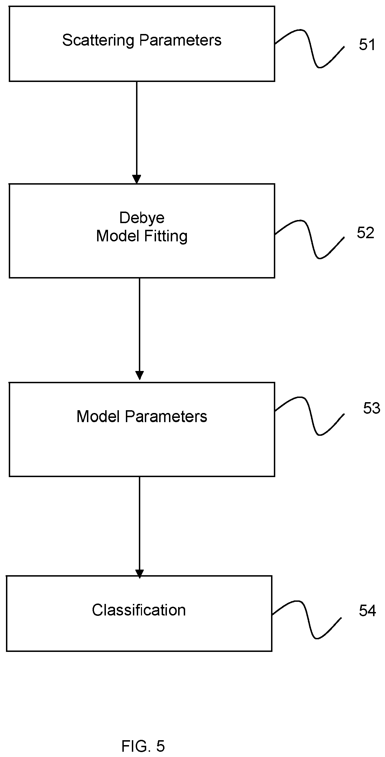

Figure 5 illustrates a data flow diagram illustrating a model-based classification method in accordance with an embodiment of the invention described herein.

As illustrated in Figure 5, the set of scattering parameters over the range of frequencies is determined for the region of interest at step 51. This corresponds to step 41 illustrated in Figure 4.

The set of scattering parameters will accordingly then be subjected to a pre-classification process in order to determine information that is representative of and/or based on the change of scattering parameters over the range of frequencies, as described with respect to step 42 of Figure 4.

In the illustrated example of Figure 5, the pre-classification processing will include two steps, a model fitting step (step 52) and a model parameter determination step (step 53).

At step 52, a model is fitted to the scattering parameters over the range of frequencies at step 52. The model may be any suitable and desired model that can be fitted to the change of scattering parameters over the range of frequencies or that can be fitted to data that has been derived from the change of scattering parameters over the range of frequencies. The model may be a dispersion model or a geometry model, as appropriate.

In the specific example of Figure 5, a Debye model of the frequency-dependent permittivity for biological tissues in the low microwave band 0.5-20GHz is fitted to the change of permittivity with frequency. To do this, the scattering parameters over the range of frequencies are first transformed to appropriate permittivity values.

The frequency dependent scattering parameter at a boundary (between the tissue of the body part in general and the tissue of the region of interest) is defined as:

$$S(\omega) = \frac{Z_2(\omega) - Z_1(\omega)}{Z_2(\omega) + Z_1(\omega)}$$

Where $Z_1(\omega)$ is the frequency dependent impedance on the incident side of the boundary (e.g. the impedance of the body part in general) and $Z_2(\omega)$ is the impedance on the transmitting side of the boundary (i.e. the impedance of the region of interest).

The impedance is related to permittivity through:

$$Z = \sqrt{\frac{\mu}{\varepsilon}}$$

Where $\mu$ is permeability and $\varepsilon$ is the complex permittivity.

It can be assumed for biological tissues that $\mu_1 = \mu_2 = \mu$, where $\mu_0 = 4\pi \times 10^{-7}$ H/m is the permeability of free space. With this assumption the equation for 5s can be rewritten as:

$$S = \frac{\sqrt{\varepsilon_1(\omega)} - \sqrt{\varepsilon_2(\omega)}}{\sqrt{\varepsilon_1(\omega)} + \sqrt{\varepsilon_2(\omega)}}$$

where $\varepsilon_1(\omega)$ is the frequency dependent permittivity on the incident side of the boundary and $\varepsilon_2(\omega)$ is the permittivity on the transmitting side of the boundary. Where the region of interest is a region within a breast, the reference permittivity $\varepsilon_1(\omega)$ is the permittivity of the breast, which can be approximated.

Making $\varepsilon_2(\omega)$ the subject:

$$\varepsilon_2(\omega) = \left[\sqrt{\varepsilon_1(\omega)}\frac{1 - S(\omega)}{1 + S(\omega)}\right]^{\frac{1}{2}}$$

In this way, the frequency dependent permittivity on the transmitting side of the boundary can be calculated from the scattering parameter(s) over the range of frequencies. The resulting set of permittivity values over the range of frequencies can then be used to fit a Debye model to the data.

In particular, a single pole Debye model for the low microwave band (0.5-20GHz) can be used to model the calculated frequency dependent permittivity for the region of interest. This model is given as:

$$\varepsilon_{Debye}(\omega) = \varepsilon_\infty + \frac{\Delta\varepsilon}{1 + j\omega\tau} + \frac{\sigma_s}{j\omega\varepsilon_0}$$

where $\varepsilon_\infty$ is the relative permittivity at infinite frequency, $\varepsilon_0 = 8.854 \times 10^{-12}$ F/m is the permittivity of free space, $\Delta\varepsilon$ is the polarisation intensity, $\omega$ is the angular frequency, $\tau$ is the time constant and $\sigma_s$ is the static conductivity.

It was shown by Lazebnik et al (Phys Med Biol. 2007 Oct 21;52(20):6093-6115. A large-scale study of the ultrawideband microwave dielectric properties of normal, benign and malignant breast tissues obtained from cancer surgeries) that tissues with high values of $\Delta\varepsilon$ and $\sigma_s$ are more likely to be cancerous. Therefore, the polarisation intensity and static conductivity can be used to classify tissue in a region of interest as either malignant or benign.

After the model has been fitted to the scattering parameters for the region of interest at step 52, the values of the parameters, $\Delta\varepsilon$ and $\sigma_s$, are derived directly from the Debye model, at step 53.

At step 54, the derived values of $\Delta\varepsilon$ and $\sigma_s$ can be used to assess the likelihood that the tissue in the region of interest is cancerous (i.e. to classify the tissue in the region of interest).

The assessment could take the form of a comparison between the derived values of $\Delta\varepsilon$ and/or $\sigma_s$ and a linear or non-linear classifier trained on known, existing data. Alternatively, a "likelihood of cancer" probability value could be provided based on the $\Delta\varepsilon$ and $\sigma_s$ values.

In an embodiment, a classifier is trained (predetermined) by performing the above model fitting method on scattering parameter data obtained (using the method of Figure 3) from sample lesions with known, histological diagnosis as being malignant (Ca) or benign (Cyst (Cy)).

It will be appreciated here that any suitable model may be used instead of the Debye model. For example, the change in the permittivity (i.e. the change in $\varepsilon_2(\omega)$ with frequency may be modelled using a single pole Drude model of permittivity for biological tissues in the low microwave band (0.5-20GHz):

$$\varepsilon(\omega) = \varepsilon_\infty \varepsilon_0 + \frac{\Delta\varepsilon\,\varepsilon_0}{1 + (j\omega\tau)^{1-\alpha}} + \frac{\sigma_s}{j\omega}$$

where $\varepsilon_\infty$ is the relative permittivity at infinite frequency, $\varepsilon_0$ is the permittivity of free space, $\Delta\varepsilon$ is the polarisation intensity, $\omega$ is the angular frequency, $\tau$ is the time constant, a is an exponent parameter, which takes a value between 0 and 1, allowing the description of different spectral shapes and $\sigma_s$ is the static conductivity contribution.

Furthermore, it will be appreciated that although the embodiment of Figure 5 has been described with respect to fitting a model to the determined scattering parameters over the entire range of frequency, this is not required. In embodiments, the scattering parameters may be subjected to an earlier processing step in advance of the model fitting. For example, the scattering parameters may be subjected to one or more feature reduction processes, as is described below, and the model may accordingly be fitted to such a processed set of scattering parameters. In particular, the model may be fitted to a subset of frequencies comprising some but not all of the overall range of frequencies. Alternatively, the range of frequencies may be divided into a plurality of bands of frequencies and a model may be fitted to the average values of the scattering parameters (or corresponding permittivity values) for respective bands of frequencies, as desired. The bands of frequencies used in such an arrangement may be selected based on a principal component analysis of the scattering parameters over the range of frequencies.

**Feature reduction classifiers**

The Applicants have recognised that in some cases, the medical imaging system 2 of the invention will have recorded more data (or rather more complex data) than is required to accurately classify a region of interest. For example, in some cases the system may derive scattering parameter data for more bands or ranges of frequencies than are actually needed to accurately classify a region of interest.

The Applicants have also recognised that in such cases it may be desirable to first reduce the complexity of the data (e.g. to reduce the time and storage space required) while minimising any loss of information essential to the analysis. In embodiments, therefore, the scattering parameter data over the range of frequencies will undergo a process of "feature reduction", which is essentially a method of compressing the features (feature space) of the scattering parameter data to the most informative features for tissue classification.

The reduced feature scattering parameter data (which has increased separability) for a region of interest can then be analysed to classify the region of interest. Specifically, the reduced feature scattering parameter data over the range of frequencies for a region of interest may be compared to a corresponding classifier derived from scattering parameter data (detected for earlier samples of known tissue types) which has undergone a corresponding process of feature reduction.

Accordingly, the pre-processing step 42 in Figure 4 will, in embodiments, comprise performing a feature reduction process on the scattering parameters obtained over the range of frequencies for the region of interest. Such feature reduction processes may be used in combination with other pre-processing methods, such as the model fitting method described above, or may be used separately in alternative arrangements, depending on the type of classifier to be used in step 43 of Figure 4 to classify the tissue in the region of interest.

Various feature reduction processes in accordance with embodiments of the present invention will now be described with respect to the type of classifier to be used to classify the tissue in the region of interest.

### Bandwidth averaging

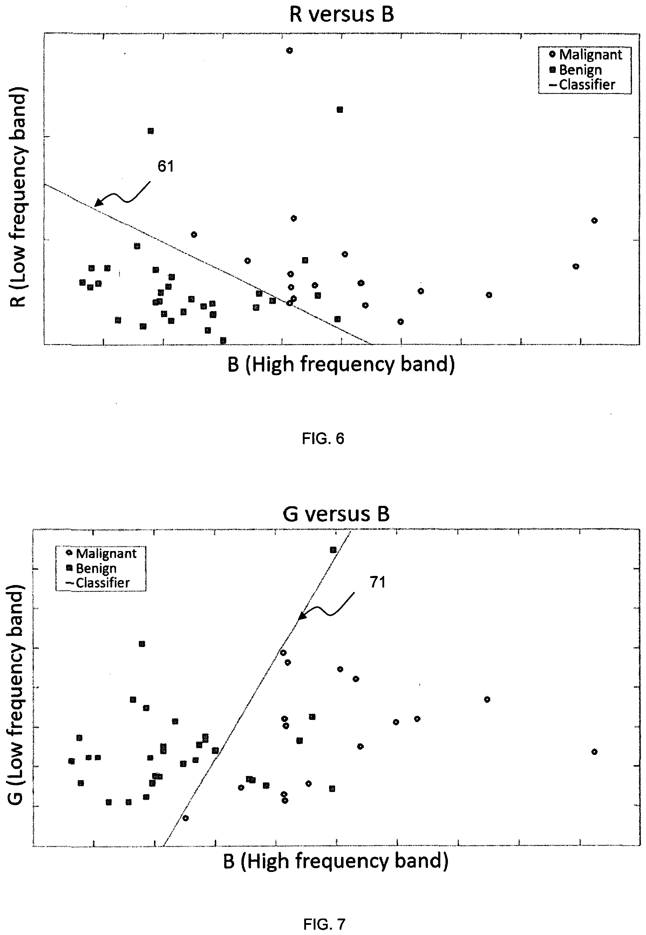

In accordance with the embodiment illustrated in Figures 6 and 7, the processor 4 will perform a feature reduction process on the scattering parameters pre-classification by dividing the range of frequencies into a plurality of bands and determine an average magnitude value of the scattering parameters for the region of interest for respective bands of frequency.

The bands may be selected in any suitable or desired manner. For example, the bands may be selected manually. However, in embodiments the two or more bands are selected by performing a feature selection process, such as a genetic algorithm, to automatically select the most suitable or relevant bands for the classification process.

After the feature reduction process, the processor 4 classifies the region of interest based on an average magnitude value of the scattering parameters for the region of interest over a first frequency range (a first band) and on an average magnitude value of the scattering parameters for the region of interest over a second frequency range (a second band).

To facilitate this, the present invention may determine a classifier which allows new samples (which have not been subjected to separate analysis) to be classified as, e.g., either malignant or benign, based on the sample's average magnitude response for the different frequency bands.

In the example of Figures 6 and 7, the data from a study performed based on 48 patients exhibiting unifocal lesions with a histological diagnosis as being malignant (Ca) or benign (Fibroadenoma (Fa) and Cyst (Cy)) was used to determine a classifier for identifying the type of tissue in the region of interest based on the change of scattering parameters over the range of frequency. The evaluated studies comprised 17 cases with malignant diagnosis and 31 cases with benign diagnosis (12 Fa and 19 Cy).

The radio frequency response was recorded for the region of interest of each patient over the 3 to 8 GHz range. To characterize the response with a limited number of parameters, the 3 to 8 GHz range was split into three bands: the low frequency band (from 3 to 4.6 GHz), the medium frequency band (from 4.7 to 6.3 GHz) and the high frequency band (from 6.4 to 8.9 GHz), where the three bands used in this example have been selected manually. Then, the average magnitude response for each band (labelled as R, G and B for low, medium and high frequencies, respectively) was computed.

The differences between the RF properties of the malignant and benign lesions were analysed using a set of scatter plots of different combinations of R, G and B values.

Figures 6 and 7 show examples of such scatter plots. Specifically, Figure 6 shows a plot of R versus B, whereas Figure 7 shows a plot of G versus B. In this regard, it will be appreciated that the selection of different combinations of RGB frequency bands corresponds to a manual feature selection, although an automatic feature selection method could have been used.

As shown, the samples may be used to derive a linear classifier 61, 71 which allows new samples (which have not been subjected to separate analysis) to be classified as either malignant or benign based on the sample's average magnitude response for the different frequency bands. The linear classifier 61, 71 defines an optimised decision boundary (which in this case corresponds to a straight line) which divides the plot into malignant and benign classes. Any known linear discriminant method may be used for deriving a linear classifier 61, 71 based on the data, such as Linear Discriminant Analysis (LDA), logistic regression etc.

As can be observed, the different plots show different degrees of discrimination of malignant from benign cases. Specifically, it has been found that the most accurate classification is provided by the R versus B plot (i.e., High against Low frequency magnitude), which allows to discriminate malignant from benign cases with a positive predicted value (PPV) of 76%, followed by the G vs B plot (i.e., Medium against Low frequency magnitude), with a PPV of 73%. The use of the R versus B plot may provide a more accurate classification as the frequency ranges are non-adjacent.

It will be appreciated here that although Figures 6 and 7 have been described with respect to deriving a linear classifier, this is not required. It is possible, for example, for the samples to be used to derive a non-linear classifier which allows new samples (which have not been subjected to separate analysis) to be classified as either malignant or benign based on the sample's average magnitude response for the different frequency bands. The non-linear classifier may define a more optimised decision boundary (compared to that of a straight line) which divides the plot into malignant and benign classes. Any known non-linear discriminant method may be used for deriving a non-linear classifier, such as a support vector machine or neural network method, etc.

### Multivariant analysis

The Applicants have recognised that it is possible to achieve feature reduction by performing a multivariant analysis during the pre-classification process described above with respect to Figure 4.

In general, multivariant analysis involves projecting scattering parameters to a different coordinate space in which features relating to the change of scattering parameters with frequency have greater separability (e.g. compared to their separability in the original space within which the scattering parameters are defined). These features can then be compared against a classifier (defined for that coordinate space) to classify or identify a tissue in a region of interest.

Any suitable or desired multivariant analysis method may be used, such as independent component analysis (ICA).

In an embodiment, however, multivariant analysis comprises performing a principle component analysis (PCA) to transform the scattering data to a new set of axes. In particular, principal component analysis will include performing an orthogonal transformation on the set of scattering parameters derived for the region of interest over the range of frequencies to convert the set of scattering parameters into a set of values representing variables called principal components. The number of distinct principal components is equal to the number of original features, i.e. the number of frequency points over the range of frequencies.

In an embodiment of the present invention, a classifier is trained by performing PCA on the scattering parameters obtained (using the method of Figure 3) from a study of a number, m, of lesion samples with a known, histological diagnosis as being malignant (Ca) or benign (Cyst (Cy)). In particular, (m=) 37 lesion samples comprising 18 cases with a malignant diagnosis and 19 cases with a benign diagnosis are used. The scattering parameters for this study were obtained by stepping the frequency over 101 discrete frequency pulses over the frequency range 3-8 GHz.

The scattering parameters from all 37 lesion samples may be put together into a single matrix, A, where each row in the matrix corresponds to the scattering parameters for a given lesion sample.

The principal components are then determined from the scattering parameter matrix A by first calculating the covariance matrix, C, which is indicative of the extent to which the frequencies correlate to each other. The covariance matrix C is given by:

$$C = (transpose(A-mean(A))(A-mean(A)))/m$$

Then, the eigenvectors, V, and corresponding eigenvalues, D, of the covariance matrix are calculated by determining the solution of the following equation:

$$CV = VD$$

Finding the eigenvectors V and eigenvalues D of the covariance matrix C can be thought of as fitting an n-dimensional ellipsoid (where n corresponds to the number of original features) to the variance of the data, where each axis of the ellipsoid represents a principal component.

Each eigenvector (which is mutually orthogonal to the other eigenvectors) can then be used as an axis of the coordinate space to which the scattering parameters are projected. In particular, an eigenvector, $v_i$ in V, can be seen as an axis of a coordinate system that the scattering parameter data, S, can be projected onto, where the value, $S'_i$, is a scattering parameter projected (i.e. linearly transformed) onto the given eigenvector $v_i$ axis:

$$S'_i = Sv_i$$

$$S'_i = S_1v_{i,1} + S_2v_{i,2} + \dots S_nv_{i,n}$$

Thus $S'_i$ corresponds to the distance from the origin of the coordinate system along the direction of the $i^{th}$ principal component. The value of this distance therefore relates to the correlation of the variance described by its associated principal component.

The appropriately transformed/projected scattering parameters over the range of frequencies can then be subjected to a discriminant analysis method to determine a classifier.

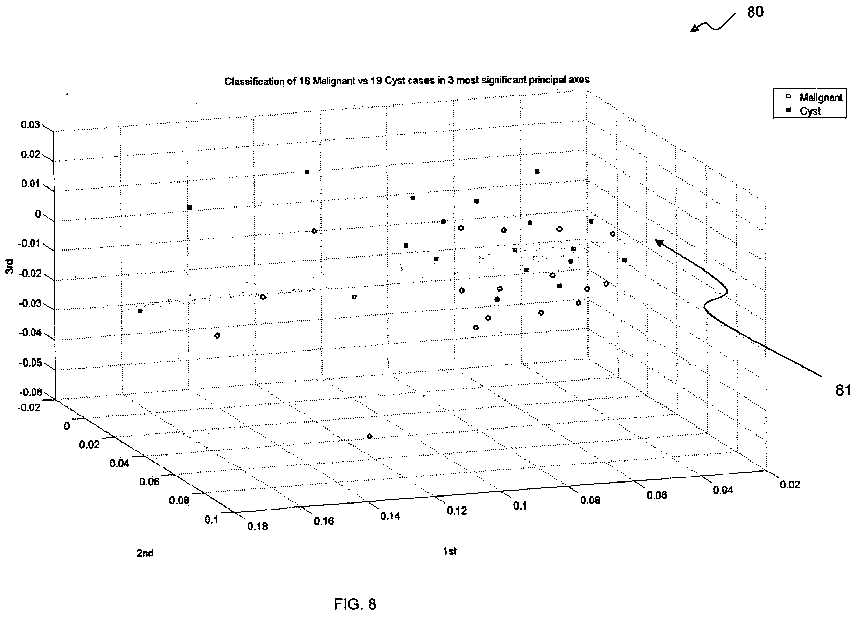

Figure 8 demonstrates the results of performing PCA on the scattering parameters obtained for the 37 lesion samples mentioned above by stepping the frequency over 101 discrete frequency pulses over the frequency range 3-8 GHz.

For ease of explanation and illustration, Figure 8 shows a co-ordinate system 80 having only three-dimensions having axes corresponding to the first, second and third principal components (i.e. the three most significant principal components having the largest variance). However, it will be appreciated that any number of dimensions (or principal components) can be used in the classification process. The maximum number of principle components is limited to a value equal to the number of dimensions in the original scattering parameter data.

As shown in the example of Figure 8, a Linear Discriminant Analysis (LDA) method was adopted to find a hyper plane 81 that best separates the malignant and benign tissue types based on the samples' position within the projected space. Any known linear discriminant method may be used for deriving a linear classifier based on the data, such as Linear Discriminant Analysis (LDA), logistic regression etc. Alternatively, a non-linear classifier may be used to define a more optimised decision boundary (compared to that of a plane). Any suitable non-linear discriminant method may be used to determine a non-linear classifier, such as a support vector machine method or a neural network method, etc.

By determining a hyperplane 81 using the three most significant principal components, a positive predicted value (PPV) of 68% is achieved. It was also found that by increasing the number of principal axes (and thus the dimensions of the coordinate space to which the scattering parameters are projected) to 10, a PPV of 81% is achieved. The classifier may be trained based on scattering parameters projected to a coordinate space having any number of principal components.

Using the above projection method, new samples (which have not been subjected to separate analysis) can be compared to the determined hyperplane 81 to be classified as either malignant or benign.

For example, the scattering parameters for the region of interest may be projected to the coordinate space (a reference coordinate space) that has been derived based on a principal component analysis of scattering parameters determined for the 37 lesion samples mentioned above, and the region of interest may be classified based on the position of the projected scattering parameters within the coordinate space.

It will be appreciated that although the embodiment of Figure 8 has been described with respect to performing PCA for the entire range of frequencies (to project the scattering parameters to a reference coordinate space), this is not required. In embodiments, the scattering parameters may undergo a pre-classification process of feature reduction and/or selection, e.g. as described above, and the processed set of scattering parameters may be used as the inputs to the PCA process (i.e. may be projected to the reference coordinate space). In particular, the scattering parameters over a subset of frequencies (including some but not all of the frequencies) may be used in the PCA (to project the scattering parameters for the subset of frequencies to the predefined reference coordinate space). Alternatively or additionally, the range of frequencies may be divided into a plurality of bands of frequencies and the average scattering parameters for respective bands of frequencies may be used as the inputs to the PCA process (i.e. may be projected to the predefined reference coordinate space), as desired.

As described, the classification of the region of interest can be made based on the scattering parameters from which the region of interest is first identified. That is, the scattering parameters derived for generating an image can be used not only to identify a region of interest, but also to identify the type of tissue in the region. Consequently, it is not necessary to obtain any additional data after determining a region of interest in order to make this classification which can be derived automatically.

It will be appreciated that the concept of the invention may be extended to encompass further sub-classification within the malignant and/or benign classes. This may be achieved using a multi-class classification.

It will also be appreciated that while the drawings have been described with respect to classifying a tissue as either malignant or benign, this is not required. The present invention may be extended to encompass classification between other types of tissue, such as to classify/identify different types of tissue (e.g. skin, muscle, fat, adipose etc.) and/or lesions (e.g. carcinoma, cysts, fibroadenoma, Lipoma). This may be done by determining ("training") a classifier using the methods described above, based on scattering parameters obtained for known tissue samples corresponding to the tissue types to be classified in subsequent samples.

To avoid unnecessary duplication of effort and repetition of text in the specification, certain features are described in relation to only one or several aspects or embodiments of the invention. However, it is to be understood that, where it is technically possible, features described in relation to any aspect or embodiment of the invention may also be used with any other aspect or embodiment of the invention.

The invention is not limited to the embodiments described herein, and may be modified or adapted without departing from the scope of the present invention.

**Claims**

1. A microwave imaging system (2) comprising:

   a microwave antenna array (6) comprising a transmitting antenna and a plurality of receiving antennas, wherein the transmitting antenna is configured to transmit microwave signals over a range of frequencies so as to illuminate a body part (36) of a patient and the receiving antennas are configured to receive the microwave signals following scattering within the body part (36) and acquire data representing the received microwave signals affected by scattering arising from objects within the body part (36); and
   a processor (4);
   **characterised in that** the processor (4) is configured to:

   process the acquired data to determine a plurality of sets of scattering parameters for a plurality of points within the body part (36), wherein each set of scattering parameters represents the scattered field, at respective frequencies across the range of frequencies, from a corresponding point within the body part (36);
   generate an output indicative of the internal structure of the body part (36), using the plurality of sets of scattering parameters, to identify a region of interest within the body part (36); and
   classify the region of interest based on a type of tissue therein by analysing the change of the scattering parameters, in a set of scattering parameters corresponding to the region of interest, over the range of frequencies.

**2.** A microwave imaging system (2) as claimed in claim 1, wherein the processor (4) classifies the region of interest based on an average magnitude value of the scattering parameters in the set of scattering parameters corresponding to the region of interest over a first frequency range and on an average magnitude value of the scattering parameters in the set of scattering parameters corresponding to the region of interest over a second frequency range.

**3.** A microwave imaging system (2) as claimed in claim 2, wherein the processor (4) classifies the region of interest based on a decision boundary dividing values for the first and second frequency ranges into at least two classes.

**4.** A microwave imaging system (2) as claimed in claim 2 or 3, wherein the first and second frequency ranges are non-adjacent.

**5.** A microwave imaging system (2) as claimed in any preceding claim, wherein:

analysing the change of the scattering parameters in the set of scattering parameters corresponding to the region of interest over the range of frequencies comprises determining a change in permittivity with frequency based on the change of the scattering parameters in the set of scattering parameters over the range of frequencies; and
the processor (4) is configured to classify the region of interest based on the determined change in permittivity with frequency.

**6.** A microwave imaging system (2) as claimed in any preceding claim, wherein:

analysing the change of the scattering parameters in the set of scattering parameters corresponding to the region of interest over the range of frequencies comprises determining a static conductivity value and/or a polarisation intensity value for the region of interest, based on the change in scattering parameters in the set of scattering parameters over the range of frequencies; and
the processor (4) is configured to classify the region of interest based on a predetermined correlation between tissue type and the static conductivity value and/or the polarisation intensity value.

**7.** A microwave imaging system (2) as claimed in any preceding claim, wherein:

the processor (4) is configured to project the scattering parameters in the set of scattering parameters corresponding to the region of interest to a predefined reference coordinate space; and
the processor (4) is configured to classify the region of interest based on the position of the projected scattering parameters within the predefined reference coordinate space.

**8.** A microwave imaging system (2) as claimed in claim 7, wherein the predefined reference coordinate space comprises a plurality of axes, wherein each axis corresponds to a principal component of a set of reference data.

**9.** A microwave imaging system (2) as claimed in claim 8, wherein the set of reference data comprises a plurality of sets of scattering parameters obtained for respective sets of sample tissues of known tissue types.

**10.** A microwave imaging system (2) as claimed in any preceding claim, wherein the processor (4) classifies the region of interest as malignant or benign.

**11.** A microwave imaging system (2) as claimed in any preceding claim, wherein the microwave antenna array is formed on a substrate, wherein the substrate is contoured or contourable to conform to the body part (36).

**12.** A microwave imaging system (2) as claimed in claim 11, wherein the substrate is hemispherical.

**13.** A microwave imaging system (2) as claimed in any preceding claim, wherein the processor is further configured to generate an image of the internal structure of the breast based on the plurality of sets of scattering parameters.

**Patentansprüche**

**1.** Mikrowellenbildgebungssystem (2), umfassend:

ein Mikrowellenantennenarray (6), umfassend eine Sendeantenne und eine Vielzahl von Empfangsantennen, wobei die Sendeantenne dafür ausgelegt ist, Mikrowellensignale über einen Frequenzbereich zu senden, zu dem Zweck, einen Körperteil (36) eines Patienten zu beleuchten, und die Empfangsantennen dafür ausgelegt sind, die Mikrowellensignale nach Streuung innerhalb des Körperteils (36) zu empfangen und Daten zu erfassen, die die empfangenen Mikrowellensignale repräsentieren, die durch Streuung aufgrund von Objekten innerhalb des Körperteils (36) beeinflusst werden; und

einen Prozessor (4);
**dadurch gekennzeichnet, dass** der Prozessor (4) für folgende Vorgänge ausgelegt ist:

Verarbeiten der erfassten Daten zum Bestimmen einer Vielzahl von Sätzen von Streuparametern für eine Vielzahl von Punkten innerhalb des Körperteils (36), wobei jeder Satz von Streuparametern das an einem entsprechenden Punkt innerhalb des Körperteils gestreute Feld bei jeweiligen Frequenzen über den Frequenzbereich (36) repräsentiert;

Erzeugen einer Ausgabe, die die innere Struktur des Körperteils (36) angibt, unter Verwendung der Vielzahl von Sätzen von Streuparametern zum Identifizieren eines Bereichs von Interesse innerhalb des Körperteils (36); und

Klassifizieren des Bereichs von Interesse auf der Grundlage einer darin befindlichen Art von Gewebe, indem die Änderung der Streuparameter in einem Satz von Streuparametern, der dem Bereich von Interesse entspricht, über den Frequenzbereich analysiert wird.

2. Mikrowellenbildgebungssystem (2) nach Anspruch 1, wobei der Prozessor (4) den Bereich von Interesse auf der Grundlage eines Durchschnittswertes der Streuparameter in dem Satz von Streuparametern, der dem Bereich von Interesse entspricht, über einen ersten Frequenzbereich und auf der Grundlage eines Durchschnittswertes der Streuparameter in dem Satz von Streuparametern, der dem Bereich von Interesse entspricht, über einen zweiten Frequenzbereich klassifiziert.

3. Mikrowellenbildgebungssystem (2) nach Anspruch 2, wobei der Prozessor (4) den Bereich von Interesse auf der Grundlage einer Entscheidungsgrenze klassifiziert, die Werte für den ersten und den zweiten Frequenzbereich in mindestens zwei Klassen unterteilt.

4. Mikrowellenbildgebungssystem (2) nach Anspruch 2 oder 3, wobei der erste und der zweite Frequenzbereich nicht benachbart sind.

5. Mikrowellenbildgebungssystem (2) nach einem der vorhergehenden Ansprüche, wobei:

das Analysieren der Änderung der Streuparameter in dem Satz von Streuparametern, der dem Bereich von Interesse über den Frequenzbereich entspricht, das Bestimmen einer Änderung der Permittivität mit der Frequenz auf der Grundlage der Änderung der Streuparameter im Satz von Streuparametern über den Frequenzbereich umfasst; und

der Prozessor (4) dafür ausgelegt ist, den Bereich von Interesse auf der Grundlage der bestimmten Änderung der Permittivität mit der Frequenz zu klassifizieren.

6. Mikrowellenbildgebungssystem (2) nach einem der vorhergehenden Ansprüche, wobei:

das Analysieren der Änderung der Streuparameter in dem Satz von Streuparametern, die dem Bereich von Interesse über den Frequenzbereich entsprechen, das Bestimmen eines Wertes der statischen Leitfähigkeit und/oder eines Polarisationsintensitätswertes für den Bereich von Interesse auf der Grundlage der Änderung der Streuparameter im Satz von Streuparametern über den Frequenzbereich umfasst; und

der Prozessor (4) dafür ausgelegt ist, den Bereich von Interesse auf der Grundlage einer vorbestimmten Korrelation zwischen Gewebeart und dem Wert der statischen Leitfähigkeit und/oder dem Polarisationsintensitätswert zu klassifizieren.

7. Mikrowellenbildgebungssystem (2) nach einem der vorhergehenden Ansprüche, wobei:

der Prozessor (4) dafür ausgelegt ist, die Streuparameter in dem Satz von Streuparametern, die dem Bereich von Interesse entsprechen, auf einen vordefinierten Referenzkoordinatenraum zu projizieren; und

der Prozessor (4) dafür ausgelegt ist, den Bereich von Interesse auf der Grundlage der Position der projizierten Streuparameter innerhalb des vordefinierten Referenzkoordinatenraums zu klassifizieren.

8. Mikrowellenbildgebungssystem (2) nach Anspruch 7, wobei der vordefinierte Referenzkoordinatenraum eine Vielzahl von Achsen umfasst, wobei jede Achse einer Hauptkomponente eines Satzes von Referenzdaten entspricht.

9. Mikrowellenbildgebungssystem (2) nach Anspruch 8, wobei der Satz von Referenzdaten eine Vielzahl von Sätzen von Streuparametern umfasst, die für jeweilige Sätze von Probengeweben bekannter Gewebearten erhalten wurden.

10. Mikrowellenbildgebungssystem (2) nach einem der vorhergehenden Ansprüche, wobei der Prozessor (4) den Bereich von Interesse als bösartig oder gutartig klassifiziert.

**11.** Mikrowellenbildgebungssystem (2) nach einem der vorhergehenden Ansprüche, wobei das Mikrowellenantennenarray auf einem Substrat ausgebildet ist, wobei das Substrat zur Anpassung an den Körperteil (36) konturiert oder konturierbar ist.

**12.** Mikrowellenbildgebungssystem (2) nach Anspruch 11, wobei das Substrat halbkugelförmig ist.

**13.** Mikrowellenbildgebungssystem (2) nach einem der vorhergehenden Ansprüche, wobei der Prozessor ferner dafür ausgelegt ist, ein Bild der inneren Struktur der Brust auf der Grundlage der Vielzahl von Sätzen von Streuparametern zu erzeugen.

**Revendications**

**1.** Système d'imagerie hyperfréquence (2) comprenant :

un réseau d'antennes hyperfréquence (6) comprenant une antenne d'émission et une pluralité d'antennes de réception, dans lequel l'antenne d'émission est configurée pour émettre des signaux hyperfréquence sur une plage de fréquences de manière à éclairer une partie de corps (36) d'un patient et les antennes de réception sont configurées pour recevoir les signaux hyperfréquence suite à leur diffusion à l'intérieur de la partie de corps (36) et pour acquérir des données représentant les signaux hyperfréquence reçus affectés par la diffusion résultant d'objets à l'intérieur de la partie de corps (36) ; et
un processeur (4) ;
**caractérisé en ce que** le processeur (4) est configuré pour :

traiter les données acquises pour déterminer une pluralité d'ensembles de paramètres de diffusion pour une pluralité de points à l'intérieur de la partie de corps (36), dans lequel chaque ensemble de paramètres de diffusion représente le champ diffusé, à des fréquences respectives sur la plage de fréquences, depuis un point correspondant à l'intérieur de la partie de corps (36) ;
générer une sortie indicative de la structure interne de la partie de corps (36), en utilisant la pluralité d'ensembles de paramètres de diffusion, pour identifier une région d'intérêt à l'intérieur de la partie de corps (36) ; et
classifier la région d'intérêt sur la base d'un type de tissu à l'intérieur en analysant la variation des paramètres de diffusion, dans un ensemble de paramètres de diffusion correspondant à la région d'intérêt, sur la plage de fréquences.

**2.** Système d'imagerie hyperfréquence (2) tel que revendiqué selon la revendication 1, dans lequel le processeur (4) classifie la région d'intérêt sur la base d'une valeur d'amplitude moyenne des paramètres de diffusion dans l'ensemble de paramètres de diffusion correspondant à la région d'intérêt sur une première plage de fréquences et sur la base d'une valeur d'amplitude moyenne des paramètres de diffusion dans l'ensemble de paramètres de diffusion correspondant à la région d'intérêt sur une seconde plage de fréquences.

**3.** Système d'imagerie hyperfréquence (2) tel que revendiqué selon la revendication 2, dans lequel le processeur (4) classifie la région d'intérêt sur la base d'une frontière décisionnelle qui divise des valeurs pour les première et seconde plages de fréquences selon au moins deux classes.

**4.** Système d'imagerie hyperfréquence (2) tel que revendiqué selon la revendication 2 ou 3, dans lequel les première et seconde plages de fréquences sont non adjacentes.

**5.** Système d'imagerie hyperfréquence (2) tel que revendiqué selon l'une quelconque des revendications précédentes, dans lequel :

l'analyse de la variation des paramètres de diffusion dans l'ensemble de paramètres de diffusion correspondant à la région d'intérêt sur la plage de fréquences comprend la détermination d'une variation de la permittivité en fonction de la fréquence sur la base de la variation des paramètres de diffusion dans l'ensemble de paramètres de diffusion sur la plage de fréquences ; et
le processeur (4) est configuré pour classifier la région d'intérêt sur la base de la variation déterminée de la permittivité en fonction de la fréquence.

**6.** Système d'imagerie hyperfréquence (2) tel que revendiqué selon l'une quelconque des revendications précédentes, dans lequel :

l'analyse de la variation des paramètres de diffusion dans l'ensemble de paramètres de diffusion correspondant à la région d'intérêt sur la plage de fréquences comprend la détermination d'une valeur de conductivité statique et/ou d'une valeur d'intensité de polarisation pour la région d'intérêt, sur la base de la variation des paramètres de diffusion dans l'ensemble de paramètres de diffusion sur la plage de fréquences ; et
le processeur (4) est configuré pour classifier la région d'intérêt sur la base d'une corrélation prédéterminée entre un type de tissu et la valeur de conductivité statique et/ou la valeur d'intensité de polarisation.

7. Système d'imagerie hyperfréquence (2) tel que revendiqué selon l'une quelconque des revendications précédentes, dans lequel :

le processeur (4) est configuré pour projeter les paramètres de diffusion dans l'ensemble de paramètres de diffusion correspondant à la région d'intérêt sur un espace prédéfini de coordonnées de référence ; et
le processeur (4) est configuré pour classifier la région d'intérêt sur la base de la position des paramètres de diffusion projetés à l'intérieur de l'espace prédéfini de coordonnées de référence.

8. Système d'imagerie hyperfréquence (2) tel que revendiqué selon la revendication 7, dans lequel l'espace prédéfini de coordonnées de référence comprend une pluralité d'axes, dans lequel chaque axe correspond à une composante principale d'un ensemble de données de référence.

9. Système d'imagerie hyperfréquence (2) tel que revendiqué selon la revendication 8, dans lequel l'ensemble de données de référence comprend une pluralité d'ensembles de paramètres de diffusion obtenus pour des ensembles respectifs de tissus échantillons de types de tissu connus.

10. Système d'imagerie hyperfréquence (2) tel que revendiqué selon l'une quelconque des revendications précédentes, dans lequel le processeur (4) classifie la région d'intérêt comme étant maligne ou bénigne.

11. Système d'imagerie hyperfréquence (2) tel que revendiqué selon l'une quelconque des revendications précédentes, dans lequel le réseau d'antennes hyperfréquence est formé sur un substrat, dans lequel le substrat est profilé ou peut être profilé pour qu'il soit adapté à la partie de corps (36).

12. Système d'imagerie hyperfréquence (2) tel que revendiqué selon la revendication 11, dans lequel le substrat est hémisphérique.

13. Système d'imagerie hyperfréquence (2) tel que revendiqué selon l'une quelconque des revendications précédentes, dans lequel le processeur est en outre configuré pour générer une image de la structure interne de la poitrine sur la base de la pluralité d'ensembles de paramètres de diffusion.

2

FIG. 1

36
35 30
31 8
16 18
20
24 26
28
22 4

FIG. 2

Energize Antenna *m*

Detect on antennae n= 1 ...*N*
*n ≠ m*

*m = m* + 1

*m = N*?

No

Yes

All data sets acquired?

Yes

End

No

Move Array

FIG. 3

FIG. 4

Scattering Parameters — 51

Debye
Model Fitting — 52

Model Parameters — 53

Classification — 54

FIG. 5

## R versus B

FIG. 6

## G versus B

FIG. 7

Classification of 18 Malignant vs 19 Cyst cases in 3 most significant principal axes

FIG. 8

EP 3 554 339 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016106334 A
- US 2006241409 A
- US 2012190977 A
- WO 2009060181 A

**Non-patent literature cited in the description**

- **LAZEBNIK et al.** *Phys Med Biol.,* 21 October 2007, vol. 52 (20), 6093-6115